# EUROPEAN PATENT APPLICATION

(11) **EP 4 485 043 A1**
(43) Date of publication of application: **01.01.2025**
(21) Application number: 23182391.5
(22) Date of filing: 29.06.2023
(51) Int. Cl.: G02B 27/01, A61B 34/00, G06T 19/00, H04N 13/30

(54) **DISPLAY SYSTEM, MEDICAL SYSTEM WITH A DISPLAY SYSTEM AND METHOD OF DISPLAYING**

(71) Applicant: Siemens Healthineers AG, 91301 Forchheim (DE)
(72) Inventor: Pelanis, Egidijus, 0176 Oslo (NO)
(74) Representative: Siemens Healthineers Patent Attorneys

(57) **Abstract**

At least one aspect of the invention is related to a display system for displaying medical images based on three-dimensional image data of an object superimposed on the object to be viewed by a person, comprising a display unit located between the person and the object, the display unit comprising an at least partially transparent screen, a first array of convex lenticular lenses optically coupled to the screen and a second array of convex lenticular lenses optically coupled to the screen, whereby the first array is mounted to the front side of the transparent screen and the second array is mounted to the back side of the transparent screen, whereby the convex side of the lenses of the second array is aligned in an opposite direction to the convex side of the lenses of the first array, a tracking system adapted to provide information indicative of the relative positions and/or relative orientations of the display unit, the object and at least one eye of the person to one another, and a control unit adapted to control the display system to three-dimensionally display at least one medical image of the object on the screen in such a way that the medical image coincides anatomically correct to the object when viewed by the person.

## Description

In one aspect the invention relates to a display system for displaying three-dimensional medical image data of an object superimposed on the object to be viewed by a user. In a further aspect the invention relates to a method of displaying at least one three-dimensional medical image of an object superimposed on the object with a display system.

In hospitals, especially during medical interventions or minimal invasive surgery with image-based surveillance and/or navigation, it is crucial to keep an overview of the procedure on the patient, of medical images of the field of view inside the patient presented on a display and of other information concerning the patient (for example patient condition, the clinical workflows etc.). The person responsible, for example a surgeon executing the intervention, a nurse or a technician, switches view between the patient, a monitor or touch screen to view medical images and eventually other displays or control devices. It would be desirable to be able to view at least the patient and the medical images at the same time.

Recent developments propose that the operator uses AR (augmented reality) or VR glasses to keep an eye on the patient while watching and reviewing images of the internal structure of the patient where the procedure takes place (for example vessels, heart, liver, brain etc.). One of the problems with such devices is that every person involved needs his/her own device and if the glasses are handed over to another person a new calibration process must be executed before usage.

It is known from the publication "Projektion von Daten auf Patientenoberflächen mittels Laserprojektion zur Darstellung von eingriffsrelevanten Informationen im direkten Sichtfeld des Operateurs" (2010J10357) to project medical image data on the surface of the patient, especially in an anatomically correct way. A problem with this method is that it can be difficult to see the information on the surface of the patient.

The underlying technical problem of the invention is to provide a display system that helps to view at the same time three-dimensional medical imaging data of an object and the object itself. This problem is solved by the display system of claim 1, by the medical system of claim 9 and by the method of displaying of claim 10. The dependent claims are related to further aspects of the invention.

At least one aspect of the invention is related to a display system for displaying medical images based on three-dimensional image data of an object superimposed on the object to be viewed by a person, comprising a display unit located between the person and the object, the display unit comprising an at least partially transparent screen, a first array of convex lenticular lenses optically coupled to the screen and a second array of convex lenticular lenses optically coupled to the screen, whereby the first array is mounted to the front side of the transparent screen and the second array is mounted to the back side of the transparent screen, whereby the convex side of the lenses of the second array is aligned in an opposite direction to the convex side of the lenses of the first array, a tracking system adapted to provide information indicative of the relative positions and/or relative orientations of the display unit, the object and at least one eye of the person (preferably both eyes of the person) to one another, and a control unit adapted to control the display system to three-dimensionally display at least one medical image of the object on the screen in such a way that the medical image coincides anatomically correct to the object when viewed by the person.

The transparent screen allows the person to look through the screen to the object. The first array of convex lenticular lenses allows the person to see a very precise three-dimensional impression of the medical images on the display. The first and second array of convex lenticular lenses aligned opposite to one another allow an undistorted view on the object. The tracking system monitors the relative positions and orientations of person, display unit and object to one another. The control system provides control over the display system. The object may be a patient or part of the patient, for example a limb, head, rump or an organ. The display unit may be partially mobile (for example fixed to a movable arm, telescopic stand etc.), completely mobile or static, but is not attached to the person in a way that only the person him/herself is able to view it (like for example AR glasses).

At least one aspect of the invention helps to solve the problems described in the introduction. With a display system according to at least one aspect of the invention, a person present in the operating room can view the object and 3D medical images (and - optionally - additional information) at the same time without having to change perspective or to move. Medical images of the object's inside and especially of internal organs (as for example heart, liver, brain, lungs, blood vessels etc.) are visualized not only in a three-dimensional way but also in their precise position and orientation in relation to the object and together with the object. The 3D medical image may for example consist of one or more pre-OP images or intra-OP images, for example reconstructed cone-beam CT images, CT images or MR images. Through the display system, the person is enabled to effectively monitor or supervise medical interventions with image-based surveillance without being forced to constantly change focus. The display system enables easy control of navigation processes, guiding instruments or sensors through the patient, for example catheters or guide wires through vessels, at the same time monitoring the exact location of the instrument in relation to the patient's body.

While it is possible to simply include one eye of the person, the preferred version is to include both eyes of the person to provide an optimal 3D impression (for example stereo) for the person. The eye distance may also be included in the calculation. As an alternative to tracking the position of the eyes, tracking of the head of the person and estimating the position of the eyes with average values may be used. In addition to the eye position and orientation, other characteristics of the person's eyes, for example attention, gaze on the screen and interaction may be tracked.

According to one embodiment of the invention, the tracking system comprises at least one tracking unit, adapted to track the position and orientation of at least one of the display unit, the object and the at least one eye of the person and comprises a calculation unit adapted to calculate relative positions and orientations of the screen, the object and the at least one eye of the user to one another. The at least one tracking unit provides measurement of positions and orientations, related to a general reference system or to a reference system that is part of the display system. This helps to get precise and current measurements and avoid inaccuracies through movements or displacements. The calculation unit calculates relative positions and orientations of the display unit, the object and the at least one eye of the person to each other. Additionally, the calculation unit may use other information, for example geometrical information of size and/or form of the display unit, the object and/or the at least one eye of the person. Using the accurate relative positions and orientations, the control unit is enabled to precisely control the anatomically correct superposition of the medical image and the object on the screen.

According to another embodiment of the invention, the display system comprises a memory unit adapted to store geometrical information of the size and/or form of the display unit. Additionally, the memory unit may store geometrical information of the object, of the at least one eye of the person or of other present instruments or devices. Alternatively, the information may be stored in an external data base and retrieved if necessary.

According to another embodiment of the invention, the display system comprises an electrochromic layer arranged between the screen and the second array of lenticular lenses, the electrochromic layer adapted to change opacity in response to an electrical signal. Electrochromic layers are known in the art and can be used to change transparency. Within the display system, it may be used to support the visualization of details of the medical image or other information, for example by decreasing the level of transparency of the screen.

According to another embodiment of the invention, the lenticular lenses of the first array and the lenticular lenses of the second array are arranged in regular grids. With this, a precise 3D impression of the medical images can be created.

According to another embodiment of the invention, the tracking system comprises at least one sensor or sensor array mounted to the display unit. In particular, the tracking system comprises at least one sensor or sensor array mounted to the front side of the display unit to track the position and/or orientation of the at least one eye of the person and/or at least one sensor or sensor array mounted to the back side of the display unit to track the position and/or orientation of the object. A sensor or sensor array on the front side of the display unit (front side is the side which is viewed by the person) may be orientable toward the person to measure the eye position of the at least one eye of the person. The sensor or sensor array on the front side of the display unit may track the absolute position and orientation in space if for example the display unit is static. Fixed to the display unit it may track the relative position and orientation of the person's eyes relative to the display unit. Another sensor or sensor array on the back side of the display unit may be orientable toward the object to measure the position and orientation of the object. The sensor or sensor array on the back side of the display unit may track the absolute position and orientation in space if for example the display unit is static. Fixed to the display unit it may track the relative position and orientation of the object relative to the display unit. Sensors or sensor array may comprise at least one optical camera, for example TOF camera, 3D camera or standard camera.

According to another embodiment of the invention, the tracking system comprises at least one tracking unit mounted external to the display unit adapted to track the position and/or orientation of the display unit. The tracking unit may comprise at least one of an optical tracking unit, infrared camera tracking unit, electromagnetic tracking unit, GPS tracking unit, inertial tracking unit or other known tracking unit. Additionally, the display unit may comprise passive marker elements to be tracked by the tracking unit, for example optical tracking spheres to be tracked by one camera or multiple cameras.

According to another embodiment of the invention, the tracking system is additionally adapted to track at least one instrument or device. This process may be accomplished by one or more tracking units. The position and orientation of the instrument or device may also be tracked and relative positions and orientations may be calculated and the instrument or device may be shown on the display unit.

At least one aspect of the invention is related to a medical system with a display system, additionally comprising a medical imaging system adapted to record medical three-dimensional images of the object. The three-dimensional medical image or images which are displayed coinciding anatomically correct to the object when viewed by the person may be recorded by the medical imaging system. The medical imaging system may for example consist of or comprise an x-ray imaging system, a CT system, an MR system or an ultrasound system.

At least one aspect of the invention is related to a method of displaying at least one three-dimensional medical image of an object superimposed on the object with a display system, comprising the following steps: Receiving at least one three-dimensional medical image of the object, Receiving data indicative of the relative positions and orientations of the display unit, the object and the at least one eye of the person to one another, and controlling the display unit to three-dimensionally display the medical image of the object on the screen in such a way that the image coincides anatomically correct to the object when viewed by the person. The method offers similar advantages to the display system.

According to another embodiment of the invention, the method comprises the additional steps: Updating Controlling the display unit to display the medical image of the object on the screen in such a way that the image coincides anatomically correct to the object when viewed by the person, if one or more of the following occurs: receiving at least one updated three-dimensional medical image and receiving updated data indicative of the relative positions and orientations of the display unit, the object and the at least one eye of the person to one another. The additional steps may be executed either several or multiple times or constantly up to a stop signal by the control unit (initiated automatically or by the user). This method also enables a person moving around to view the superimposed 3d images together with the object in good quality irrespective of the position the person moves to or the angle with which the persons views the screen.

According to another embodiment of the invention, the method comprises the steps: tracking the position and orientation of at least one of the display unit, the object and the at least one eye of the person, and calculating relative positions and orientations of the screen, the object and the at least one eye of the user to one another at least partially from the tracked data.

The invention will be illustrated below with reference to the accompanying figures using example embodiments. The illustration in the figures is schematic and highly simplified and not necessarily to scale.
- FIG 1: shows an embodiment of the display system with a display unit and an object and a person viewing display and object;
- FIG 2: shows the geometrical relations between person, screen and object;
- FIG 3: shows the front side of an embodiment of the display unit;
- FIG 4: shows the back side of an embodiment of the display unit;
- FIG 5: shows an embodiment of the display system and an object on a patient table;
- FIG 6: shows an embodiment of the medical system; and
- FIG 7: shows an embodiment of a method of displaying a 3D medical image of an object superimposed on the object with a display system; and
- FIG 8: shows the method of Fig. 7 with an additional updating step.

During medical interventions or minimal invasive surgery with image-based surveillance and navigation, especially but not only with remote operation of the procedure, it is important for the operating person to keep an overview of the procedure and the patient. The display system described in the Figures helps by enabling the person to view the object and 3d images at the same time without changing focus. It also gives the person the possibility to move around and change angle of view and still be able to view a precise overlay of the 3D images and the object itself.

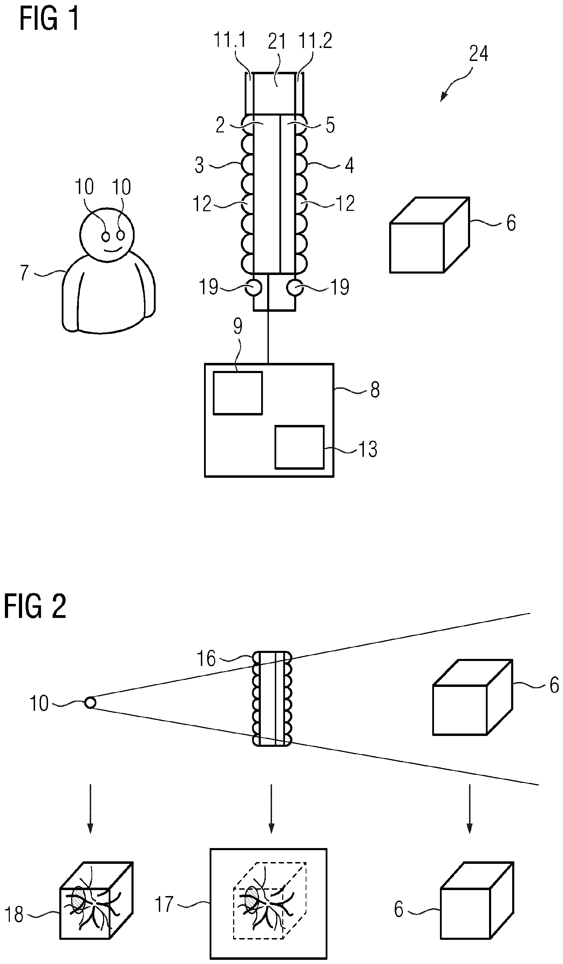

Fig. 1 shows a display system 1 for displaying medical images based on three-dimensional image data of an object 6 superimposed on the object 6 to be viewed by a person 7, the display system 1 comprising a display unit 24 and a control unit 8. The display unit 24 (shown as a cross section) is located between the person 7 and the object 6 and especially between the eyes 10 of the person 7 and the object 6. The display unit 24 comprises an at least partially and especially fully transparent screen 2 which is adapted to display images. A first array 3 of lenticular lenses 12 is mounted on the front side of and optically coupled to the transparent screen 2. The front side is meant to be the side a person is looking at.

Transparent screens to display 2D and 3D images are known in the art. The transparent screen 2 may for example work like a head up display, the display unit comprising a video generation computer to generate the image and a projector (not shown) to project the image to the transparent screen. The display unit may also work as any known 3D/volumetric display device, for example displaying light associated with at least two views (stereoview) or multiple views or as an autostereoscopic screen.

The screen 2 with the first layer 3 of lenticular lenses is adapted to three-dimensionally display 3D image data, for example reconstructed medical volume images like 3D x-ray images, CT images, 3D MR images or 3D ultrasound images. The display of 3D images on a screen with lenticular lenses is known in the art. A second array 4 of lenticular lenses 12 is mounted to the back side of the transparent screen 2 and optically coupled to the screen. The alignment of the lenses (the orientation of the convex surfaces of every single lens) of the second array 4 is in an opposite direction to the alignment of the lenses of the first array 3. This opposite orientation allows the person to have an undistorted view at the object 6 and the background (other objects, devices, patient table etc.) behind the display unit 24.

An additional electrochromic layer 5 is mounted between the transparent screen 2 and the second array 4 of lenticular lenses. The transparency of the electrochromic layer 5 can be modulated by electrical signals (for example electric current). The amount of background that is shown to the person can be reduced by using a decreased transparency. This may be used temporarily to show additional information on the screen, for example text, or it may be used to emphasize parts of the medical image or to tone down the visibility of the object.

The transparent screen 2 may be mounted in a frame 21, which surrounds the screen 2 and overlaps with it at the borders.

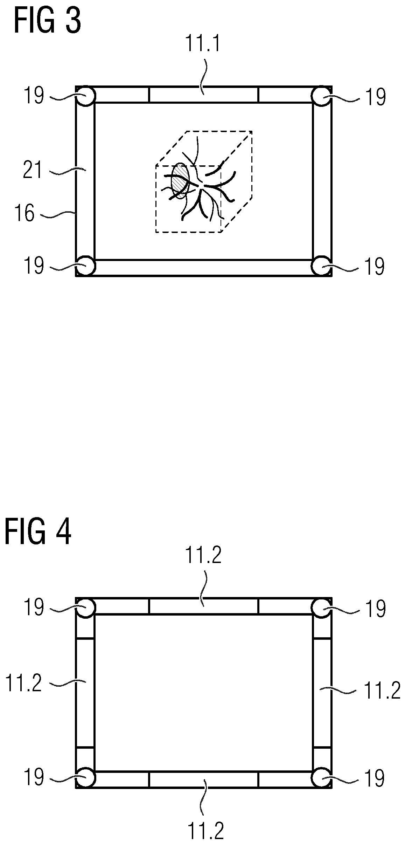

The display system 24 comprises a tracking system which may consist of at least one tracking unit or multiple tracking units with at least one sensor or sensor array each. A first sensor array 11.1 may be mounted to the front side of the display unit, especially to the frame 21 of the display unit 24 to not shadow the screen 2 - for example shown in the view onto the front side of the display unit in Fig.3. The first sensor array 11.1 may comprise cameras, for example 3D or TOF cameras, to track the position (and orientation) of the eyes 10 of the person 7. This position may be tracked as a position relative to the display unit or alternatively, for example if the display unit has a fixed position and orientation, as an absolute position. As an alternative to tracking the position of the eyes, tracking of the head of the person and estimating the position of the eyes with average values may be used. The camera or cameras of the first sensor array 11.1 may also comprise filters, for example infrared filters to view fluorescence. The first sensor array 11.1 may also comprise additional depth sensors. The first sensor array 11.1 may be adapted to generate additional references for correction of placement of the digital overlay and its focus at the correct distance. Additionally, the first sensor array 11.1 may also be adapted to track other characteristics of the person, for example attention, gaze on the screen or interaction.

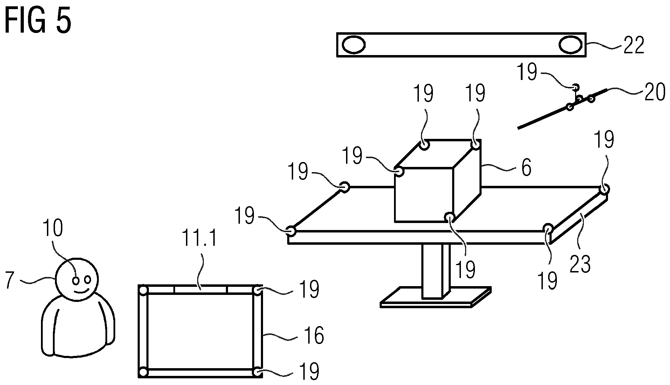

A second sensor array 11.2 may be mounted to the back side of the display unit 24, especially to the back side of the frame 21 of the display unit 24 - for example shown in the view onto the back side of the display unit in Fig.4. The second sensor array 11.2 may comprise cameras to track the object 6 (part of the patient or the patient), to measure position and orientation of the object 6. Position and orientation may be tracked relative to the display unit or alternatively as absolute position and orientation. The second sensor array 11.2 may be an advanced sensor array including different sensors, for example cameras with and without filters, IR sensors to view fluorescence, depth sensors or other sensors. The second sensor array 11.2 may also be adapted to track other devices, as for example the patient table, instruments, medical devices or parts of medical device (for example movable C-arms etc.). In addition to the first sensor array 11.1 and second sensor array 11.2, an additional external tracking unit 22 may be comprised, the external tracking unit 22 adapted to track the display unit 24 regarding its position and orientation in space (not shown in Fig.1, shown in Fig. 5). External tracking unit 22 may consist of at least one of an optical tracking unit, infrared camera tracking unit, electromagnetic tracking unit, GPS tracking unit, inertial tracking unit or other known tracking unit. Additionally, the display unit 24 may comprise multiple passive marker elements to be tracked by the external tracking unit 22, for example optical tracking spheres 19 to be tracked by one camera or multiple cameras - also shown in Figures 3 and 4 on the front and back sides of the display unit.

The display system 1 additionally comprises a control unit 8 which may be part of the display unit 24 or external to the display unit 24. An external control unit 8 is communicationally connected to the display unit 24, either by cable or by a wireless communication link (for example WiFi). Additionally, the display system may comprise a calculation unit 9 and a memory unit 13. The control unit 8 is adapted to control the display of a 3D medical image on the display unit 24 superimposed anatomically correct to the real object 6 when viewed by the person 7. The control unit makes sure of a precise placement of the overlay in terms of position and depth. For this, the control unit uses the relative positions and orientation of the display unit in relation to the object and the display unit in relation to the eyes of the person. If necessary, updates on the positions and orientations are used to update movements of the person 7, the object 6 and the display unit 24.

Fig. 2 schematically shows the geometrical relations between the eye(s) 10 of person 7, screen 2 and the object 6. The perception 18 of person 7 comprises an anatomically correct overlay of the object 6 and the medical image 17 in 3D. The screen 2 of display unit 16 displays the 3D medical image 17.

Figure 5 shows an arrangement of the display unit 16 between the person 7 and the object 6. The object 6, for example a patient or part of a patient, is located on a patient table 23. An instrument 20, for example a catheter, guide wire, needle, surgical tool, bone screw or other device, may be present to help to treat or diagnose the patient. An external tracking unit 22 is also located in the room. The external tracking unit 22 is adapted to track the position and/or orientation of the display unit 16. The external tracking unit 22 may for example be an optical tracking unit (alternatively an infrared camera tracking unit, electromagnetic tracking unit, GPS tracking unit, inertial tracking unit or other known tracking unit) with at least one camera. The display unit may comprise passive marker elements to be tracked by the external tracking unit 22, for example optical tracking spheres 19 to be tracked by the camera (or for example RFID transponders to be tracked by an RFID receiver/transmitter etc.).

The external tracking unit 22 may additionally be adapted to track the patient table 23 and/or the instrument 20 and/or other devices. For this, optical tracking spheres 19 are mounted to the patient table 23 and/or the instrument so that the position and orientation of the instrument or device may also be tracked, and relative positions and orientations may be calculated. With this, the overlay of the medical images can be displayed in its precise position and orientation and depth relative to the patient table 23 or instrument 20 or device. Additionally, for example if the instrument is also shown in the medical images, the position can be further defined relative to the instrument itself.

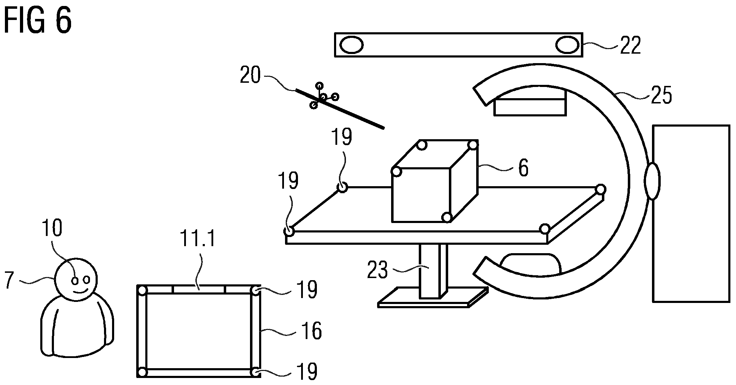

Fig. 6 shows a similar arrangement of the display unit 16 between the person 7 and the object 6 together with an x-ray imaging apparatus 25, especially an x-ray c-arm apparatus with an c-arm to which an x-ray detector and x-ray source are mounted. The x-ray apparatus 25 is adapted to acquire x-ray images of the object 6 on the patient table 23, for example a series of projection images from different angulations that can be reconstructed to a 3D volume of the object 6. The 3D or volumetric images may be displayed on the display unit in such a way that the medical image coincides anatomically correct to the object when viewed by theperson as described before.

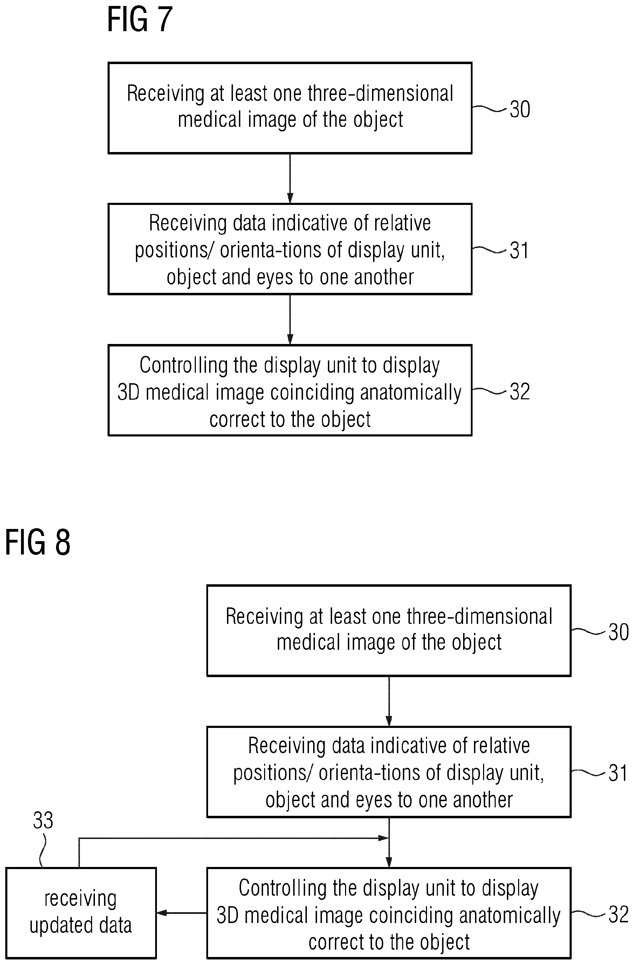

Figures 7 and 8 show steps of an embodiment of the method of displaying a 3D medical image of an object superimposed on the object with a display system. In a first step 30 at least one three-dimensional medical image of the object 6 is received in order to be displayed via the display unit. The 3D medical image may be acquired live by a medical imaging device, for example the x-ray c-arm device (for example by recording a number of projection images with different angulations and reconstructed to a 3D medical image) or may have been acquired before and stored in a storing device. In a second step 31, which may also take place before the first step 30, data indicative of the relative positions and orientations of the display unit, the object and the at least one eye of the person to one another is provided. The data may be collected by tracking units and sensors as described before. This may for example be done by tracking relative positions and orientations of object and eyes of the person relative to the display unit. It may also be done by tracking absolute positions and orientations of object, display unit and eyes of the person and calculating the relative positions and orientations of the display unit, the object and the eyes of the user to one another at least partially from the tracked data.

In a third step 32, the display unit is controlled to three-dimensionally display the 3D medical image of the object on the screen in such a way that the image coincides anatomically correct to the object when viewed by the person. To optimize the alignment, several measurements and other information, for example the position and orientation of additional devices may be used.

In a fourth step 33, displaying the 3D medical image of the object on the display unit may be updated in regard to the anatomically correct overlay if one or more of the following occurs: receiving at least one updated three-dimensional medical image and receiving updated data indicative of the relative positions and orientations of the display unit, the object and the at least one eye of the person to one another. With this, changes in the geometrical arrangement between the object, the display unit and the eyes of the person are taken into account so that the image still coincides anatomically correct to the object when viewed by the person. This may be important for example if the person moves around or moves his/her head, if the display unit is placed in a different position or orientation or if the object or patient table is moved. The update may be repeated within short time intervals or continuously.

The 3D medical images may for example consist of one or more pre-OP (acquired before operation) images or intra-OP images, for example reconstructed cone-beam CT images, CT images or MR images.

Independent of the grammatical term usage, individuals with male, female or other gender identities are included within the term.

At least one aspect of the invention is related to a display system for displaying medical images based on three-dimensional image data of an object superimposed on the object to be viewed by a person, comprising a display unit located between the person and the object, the display unit comprising an at least partially transparent screen, a first array of convex lenticular lenses optically coupled to the screen and a second array of convex lenticular lenses optically coupled to the screen, whereby the first array is mounted to the front side of the transparent screen and the second array is mounted to the back side of the transparent screen, whereby the convex side of the lenses of the second array is aligned in an opposite direction to the convex side of the lenses of the first array, a tracking system adapted to provide information indicative of the relative positions and/or relative orientations of the display unit, the object and at least one eye of the person to one another, and a control unit adapted to control the display system to three-dimensionally display at least one medical image of the object on the screen in such a way that the medical image coincides anatomically correct to the object when viewed by the person. At least one embodiment of the invention comprises a tracked transparent screen with lenticular lenses on both sides to pass reality behind the screen undistorted while overlaying digital information with depth on top.

## Claims

1. Display system for displaying medical images based on three-dimensional image data of an object superimposed on the object to be viewed by a person, comprising
• a display unit located between the person and the object, the display unit comprising an at least partially transparent screen, a first array of convex lenticular lenses optically coupled to the screen and a second array of convex lenticular lenses optically coupled to the screen, whereby the first array is mounted to the front side of the transparent screen and the second array is mounted to the back side of the transparent screen, whereby the convex side of the lenses of the second array is aligned in an opposite direction to the convex side of the lenses of the first array,
• a tracking system adapted to provide information indicative of the relative positions and/or relative orientations of the display unit, the object and at least one eye of the person to one another, and
• a control unit adapted to control the display system to three-dimensionally display at least one medical image of the object on the screen in such a way that the medical image coincides anatomically correct to the object when viewed by the person.

2. Display system according to claim 1, the tracking system comprising at least one tracking unit, adapted to track the position and orientation of at least one of the display unit, the object and the at least one eye of the person and a calculation unit adapted to calculate relative positions and orientations of the screen, the object and the at least one eye of the user to one another.

3. Display system according to claim 1 or 2, comprising a memory unit adapted to store geometrical information of the size and/or form of the display unit.

4. Display system according to one of the previous claims, comprising an electrochromic layer arranged between the screen and the second array of lenticular lenses, the electrochromic layer adapted to change opacity in response to an electrical signal.

5. Display system according to one of the previous claims, whereby the lenticular lenses of the first array and the lenticular lenses of the second array are arranged in regular grids.

6. Display system according to one of the previous claims, whereby the tracking system comprises at least one sensor or sensor array mounted to the display unit.

7. Display system according to claim 6, whereby the tracking system comprises at least one sensor or sensor array mounted to the front side of the display unit to track the position and/or orientation of the at least one eye of the person and at least one sensor or sensor array mounted to the back side of the display unit to track the position and/or orientation of the object.

8. Display system according to one of the previous claims, whereby the tracking system comprises at least one tracking unit mounted external to the display unit adapted to track the position and/or orientation of the display unit.

9. Display system according to one of the previous claims, whereby the tracking system is additionally adapted to track at least one instrument or device.

10. Display system according to one of the previous claims, the tracking system comprising at least one of a camera and an optical tracking sphere.

11. Medical system with a display system according to one of the claims 1 to 10, additionally comprising a medical imaging system adapted to record medical three-dimensional images of the object.

12. Method of displaying at least one three-dimensional medical image of an object superimposed on the object with a display system according to claims 1 to 10, with the following steps:
• Receiving at least one three-dimensional medical image of the object,
• Receiving data indicative of the relative positions and orientations of the display unit, the object and the at least one eye of the person to one another, and
• Controlling the display unit to three-dimensionally display the medical image of the object on the screen in such a way that the image coincides anatomically correct to the object when viewed by the person.

13. Method according to claim 12, with the additional steps:
• Updating Controlling the display unit to display the medical image of the object on the screen in such a way that the image coincides anatomically correct to the object when viewed by the person,
if one or more of the following occurs: receiving at least one updated three-dimensional medical image and receiving updated data indicative of the relative positions and orientations of the display unit, the object and the at least one eye of the person to one another.

14. Method according to one of claims 12 or 13, with the steps:
• tracking the position and orientation of at least one of the display unit, the object and the at least one eye of the person, and
• calculating relative positions and orientations of the screen, the object and the at least one eye of the user to one another at least partially from the tracked data.
